(19) Europäisches Patentamt European Patent Office Office européen des brevets

(11) **EP 4 656 719 A1**

(12) **EUROPEAN PATENT APPLICATION**
published in accordance with Art. 153(4) EPC

(43) Date of publication:
**03.12.2025 Bulletin 2025/49**

(21) Application number: 24747357.2

(22) Date of filing: **25.01.2024**

(51) International Patent Classification (IPC):
*C12N 9/42* (2006.01)    *A23L 2/38* (2021.01)
*A23L 7/104* (2016.01)

(52) Cooperative Patent Classification (CPC):
**A23L 2/38; A23L 7/104; C12N 9/2434**

(86) International application number:
**PCT/JP2024/002237**

(87) International publication number:
**WO 2024/158033 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.01.2023  JP 2023010263**

(71) Applicant: **Amano Enzyme Inc.**
**Nagoya-shi**
**Aichi 460-8630 (JP)**

(72) Inventor: **SAKAI Kiyota**
**Kakamigahara-shi, Gifu 509-0109 (JP)**

(74) Representative: **Mullholland, Lewis Paul**
**WP Thompson**
**44 Southampton Buildings**
**London WC2A 1AP (GB)**

(54)  **ENZYME PREPARATION**

(57)    [Problems] To provide a technique capable of increasing the amount of water-soluble dietary fiber in a plant-based liquid composition.

[Solution] The present technique provides an enzyme preparation containing two or more types of cellulases having different origins. The enzyme preparation according to the present technique can contain exo-type cellulase and endo-type cellulase. The enzyme preparation according to the present technique can be used as an agent for increasing the amount of water-soluble dietary fiber in a dietary fiber-containing liquid processed composition. The enzyme preparation according to the present technique can also be used as an agent for reducing residues in a dietary fiber-containing liquid processed composition.

EP 4 656 719 A1

## Description

Technical Field

The present technique relates to an enzyme preparation. More specifically, the present technique relates to an enzyme preparation containing two or more kinds of cellulases having different origins, an agent for increasing the amount of water-soluble dietary fiber in a dietary fiber-containing liquid processed composition containing the enzyme preparation, and an agent for reducing residues in a dietary fiber-containing liquid processed composition, as well as a dietary fiber-containing processed food and drink in which the enzyme preparation is used, a method for producing a dietary fiber-containing liquid processed composition, a method for increasing the amount of water-soluble dietary fiber in a dietary fiber-containing liquid processed composition, and a method for reducing residues in a dietary fiber-containing liquid processed composition.

Background Art

Drinks rich in nutrients such as protein have been widely enjoyed by people for a long time because they allow easy intake of nutrients. On the other hand, in recent years, due to the increase in vegetarians, allergy issues, religious reasons, and the like, drinks made from soybeans, oats, almonds, and the like, which are rich in plant-based proteins, have become widespread as substitutes for animal milks such as cow's milk. There has been an increasing need to obtain physiological effects that cannot be obtained from cow's milk from liquid compositions containing processed plant-based proteins. For example, oat milk (also called "oats milk") has been expanding its market in recent years because it contains a lot of $\beta$-glucan, which is known as a water-soluble dietary fiber.

For example, Patent Literature 1 discloses a method for preparing oat milk that is highly nutritious and has good stability, aroma, and taste by allowing $\alpha$-amylase and cellulase to act on oat flour. Patent Literature 2 discloses a method for obtaining cellooligosaccharides in high yields by allowing cellulase to act on natural cellulosic substances (natural water-insoluble fibrous substances containing cellulose). Patent Literature 3 discloses a dough composition with improved texture obtained by adding cellooligosaccharides to a plant-derived powder.

Citation List

Patent Literature

Patent Literature 1: CN107821600A
Patent Literature 2: International Publication WO 2006/011479
Patent Literature 3: Japanese Patent Application Laid-Open Publication No. 2009-095252

Summary of Invention

Technical Problem

Plant-based processed foods and drinks are highly useful in terms of nutritional value and storage stability, and are expected to be used in a variety of applications. On the other hand, the preparation of a plant-based liquid composition such as oat milk involves a filtration process, which generates residues including insoluble dietary fiber. How to reduce this residue and increase the amount of water-soluble dietary fiber has not yet been fully studied.

Therefore, the main object of the present technique is to provide a technique capable of increasing the amount of water-soluble dietary fiber in a plant-based liquid composition.

Solution to Problem

As a result of intensive research, the inventors of the present application have found that the amount of water-soluble dietary fiber in a plant-based liquid composition can be significantly increased by allowing two or more types of cellulase having different origins to act on a plant material. Based on this finding, the present technique has been completed through further research.

That is, the present technique first provides an enzyme preparation containing two or more types of cellulase having different origins.

The enzyme preparation according to the present technique may contain exo-type cellulase and endo-type cellulase.

As the exo-type cellulase that can be used in the enzyme preparation according to the present technique, cellulase derived from a microorganism of the genus Trichoderma can be used.

As the endo-type cellulase used in the enzyme preparation according to the present technique, cellulase derived from a microorganism of the genus Aspergillus can be used.

The enzyme preparation according to the present technique can be used as an agent for increasing the amount of water-soluble dietary fiber in a dietary fiber-containing liquid processed composition.

The increasing agent according to the present technique can contain exo-type cellulase and endo-type cellulase. In this case, the activity ratio of the exo-type cellulase to the endo-type cellulase can be set to exo-type cellulase activity:endo-type cellulase activity = 1:99 to 99:1.

The enzyme preparation according to the present technique can also be used as an agent for reducing residues in a dietary fiber-containing liquid processed composition.

The residue-reducing agent according to the present technique can contain exo-type cellulase and endo-type cellulase. In this case, the activity ratio of the exo-type cellulase to the endo-type cellulase can be set to exo-type cellulase activity:endo-type cellulase activity = 1:99 to 99:1.

The enzyme preparation according to the present technique can be used in the production of dietary fiber-containing processed foods and drinks.

Next, the present technique provides a method for producing a dietary fiber-containing liquid processed composition, which includes a cellulase treatment step of treating a dietary fiber-containing composition with two or more types of cellulase having different origins.

The dietary fiber-containing composition used in the production method according to the present technique may be a grain.

The two or more types of cellulases having different origins used in the production method according to the present technique may contain exo-type cellulase and endo-type cellulase.

As the exo-type cellulase that can be used in the production method according to the present technique, cellulase derived from a microorganism of the genus Trichoderma can be used.

As the endo-type cellulase that can be used in the production method according to the present technique, cellulase derived from a microorganism of the genus Aspergillus can be used.

In the cellulase treatment step of the production method according to the present technique, the exo-type cellulase and the endo-type cellulase can be allowed to act in an activity ratio of exo-type cellulase activity:endo-type cellulase activity = 1:99 to 99:1.

The present technique further provides a method for increasing the amount of water-soluble dietary fiber in a dietary fiber-containing liquid processed composition, and a method for reducing residues in a dietary fiber-containing liquid processed composition, the method including a cellulase treatment step of treating the dietary fiber-containing liquid processed composition with two or more types of cellulase having different origins.

Advantageous Effects of Invention

According to the present technique, it is possible to increase the amount of water-soluble dietary fiber in a plant-based liquid composition.

Description of Embodiments

A preferred embodiment for carrying out the present technique will be described below. Note that the embodiment described below shows an example of a typical embodiment of the present technique, and the scope of the present technique is not to be interpreted narrowly by this.

1. Enzyme preparation

The enzyme preparation according to the present technique contains two or more types of cellulases having different origins. As will be demonstrated in the examples described below, the present technique has succeeded in synergistically increasing the amount of water-soluble dietary fiber in the dietary fiber-containing liquid processed composition produced, more than a person skilled in the art would expect, and synergistically reducing the amount of residues, more than a person skilled in the art would expect, by treating the dietary fiber-containing composition with two or more types of cellulases having different origins.

Dietary fiber is a polymeric substance of polysaccharides and polysaccharide derivatives, and is a difficult-to-digest component in food that cannot be digested by human digestive enzymes.

Water-soluble dietary fiber is easily dissolved in water-soluble solvents but is difficult to digest with human digestive enzymes, and specific examples include resistant dextrin, inulin, polydextrose, resistant glucan, isomaltodextrin, guar gum hydrolyzate, cellooligosaccharide, etc. Cellooligosaccharide is an oligosaccharide having a structure in which about 1 to 6 glucopyranose units are bonded in a $\beta$-1,4 bond, and is a general term for cellobiose, cellotriose, cellotetraose, cellopentaose, and cellohexaose. In recent years, the physiological functions of cellooligosaccharide, like other oligosaccharides, are becoming clear, and it is expected to be a new material for functional foods.

Cellulase is an enzyme that hydrolyzes the glucosidic bonds of $\beta$-1,4 glucan. The cellulase that can be used in the present technique may be an enzyme that further has other functions as long as it has cellulase activity.

Cellulases are classified into exo-type, which decomposes cellulose molecular chains in cellobiose units in sequence from the non-reducing end, and endo-type, which randomly cleaves cellulose molecular chains from the inside. In the present technique, either exo-type cellulase or endo-type cellulase can be used, and it is preferable to use exo-type cellulase and endo-type cellulase in combination.

In the present technique, the exo-type cellulase may be an enzyme having the action of endo-type cellulase, so long as the exo-type cellulase activity is the main activity. Similarly, in the present technique, the endo-type cellulase may be an enzyme having the action of exo-type cellulase, so long as the endo-type cellulase activity is the main activity.

The origin of cellulase that can be used in the present technique is not particularly limited as long as it does not impair the action and effect of the present technique. For example, cellulase derived from microorganisms of the genus Trichoderma and cellulase derived from microorganisms of the genus Aspergillus can be mentioned.

Cellulases derived from microorganisms of the genus Trichoderma contain a large amount of exo-type cellulases (American Chemical Society, June 1, 1979, p. 237-260). Therefore, in the present technique, cellulases derived from microorganisms of the genus Trichoderma can be suitably used as the exo-type cellulases.

Cellulases derived from microorganisms of the genus Aspergillus contain a large amount of endo-type cellulases (Acta Cryst. (2002). D58, 660-667). Therefore, in the present technique, cellulases derived from microorganisms of the genus Aspergillus can be suitably used as the endo-type cellulases.

The microorganisms of the genus Trichoderma include Trichoderma reesei and Trichoderma viride. In the present technique, it is preferable to use cellulase derived from Trichoderma viride.

The microorganisms of the genus Aspergillus include Aspergillus niger, Aspergillus oryzae, Aspergillus sojae, Aspergillus saitoi, Aspergillus awamori, and Aspergillus flavus. In the present technique, it is preferable to use cellulase derived from Aspergillus niger.

In this technique, not only natural (wild-type) cellulase but also recombinant cellulase can be used. Commercially available cellulases or cellulase preparations can also be used. Examples of commercially available cellulases or cellulase preparations include cellulases derived from Trichoderma viride manufactured by Amano Enzyme Inc. as cellulases derived from microorganisms of the genus Trichoderma, and cellulases derived from Aspergillus niger manufactured by Amano Enzyme Inc. as cellulases derived from microorganisms of the genus Aspergillus.

The cellulase used in the present technique can be prepared from the culture solution of the microorganism from which the above-mentioned cellulase is derived. Specific preparation methods include a method of recovering cellulase from the above-mentioned culture or bacterial bodies of the microorganism. For example, when a cellulase-secreting microorganism is used, the bacterial bodies can be recovered from the culture solution by filtration, centrifugation, or the like as necessary, and then the enzyme can be separated and/or purified. In contrast, when a cellulase-non-secreting microorganism is used, the bacterial bodies may be recovered from the culture solution in advance as necessary, and then the bacterial bodies can be crushed by pressure treatment, ultrasonic treatment, or the like to expose the enzyme, after which the enzyme can be separated and/or purified. As a method for separating and/or purifying the enzyme, a known protein separation and/or purification method can be used without any particular limitation, and examples of the method include centrifugation, UF concentration, salting out, and various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

When exo-type cellulase and endo-type cellulase are used in combination, their blending ratio is not particularly limited and can be set appropriately depending on the origin of the cellulase used, the type of raw material, etc. The activity ratio of exo-type cellulase to endo-type cellulase can be set, for example, to exo-type cellulase activity:endo-type cellulase activity = 1:99 to 99:1, preferably 2:98 to 90:10, 3:97 to 20:80, 4:96 to 30:70, 5:95 to 40:60, 10:90 to 40:60, 20:80 to 40:60, and more preferably 30:70 to 40:60.

Regarding the activity of cellulase, the amount of enzyme that causes an increase in reducing power equivalent to 1 $\mu$mole of glucose per minute is defined as 1 unit (1 U). In this technique, the activity of cellulase is a value measured by the cellulase activity measurement method described in the Examples below.

When exo-type cellulase is used, the amount of exo-type cellulase to be added is not particularly limited and can be set appropriately depending on the origin of the cellulase used, the type of raw material, etc. The lower limit of the amount of exo-type cellulase to be added per 1 g of dietary fiber to be treated with the enzyme can be set to, for example, 0.1 U or more, preferably 0.3 U or more, 0.6 U or more, 1 U or more, 1.5 U or more, 2 U or more, more preferably 3 U or more, 6 U or more, and even more preferably 9 U or more.

The upper limit of the amount of exo-type cellulase to be added can be set, for example, to 200 U or less, 100 U or less, 50 U or less, 30 U or less, preferably 20 U or less, 15 U or less, more preferably 13 U or less, and even more preferably 11 U or less.

When endo-type cellulase is used, the amount of the endo-type cellulase to be added is not particularly limited and can be appropriately set depending on the origin of the cellulase to be used, the type of raw material, etc. The lower limit of the amount of the endo-type cellulase to be added per 1 g of dietary fiber to be treated with the enzyme can be set to, for example, 1 U or more, preferably 2 U or more, 3 U or more, 5 U or more, more preferably 10 U or more, and even more preferably 15 U or more.

The upper limit of the amount of endo-type cellulase to be added can be set, for example, to 500 U or less, 300 U or less, 200 U or less, preferably 150 U or less, 120 U or less, more preferably 100 U or less, 80 U or less, and even more preferably 60 U or less, 40 U or less, 20 U or less.

The present technique contains two or more types of cellulase having different origins, and other enzymes and other components can be used in combination as long as the action and effect of the present technique are not impaired. Examples of other enzymes include $\alpha$-amylase. Examples of other components that can be used include excipients, pH adjusters, colorants, flavoring agents, disintegrants, lubricants, stabilizers, and other components that are normally used in formulations. Furthermore, components having known or future functions can also be used in combination as appropriate depending on the purpose.

2. Agents for increasing the amount of water-soluble dietary fiber in dietary fiber-containing liquid processed compositions and agents for reducing residues

The enzyme preparation according to the present technique can be suitably used as an agent for increasing the amount of water-soluble dietary fiber in a dietary fiber-containing liquid processed composition, or as an agent for reducing residues in a dietary fiber-containing liquid processed composition.

The dietary fiber-containing composition for which the amount of water-soluble dietary fiber is to be increased and the dietary fiber-containing composition for which the residue is to be reduced are not particularly limited as long as they do not impair the action and effect of the present technique. Examples of dietary fiber-containing compositions that can be used in the present invention include liquid compositions, powder compositions, slurry compositions, and structured compositions. Specific examples of dietary fiber-containing liquid compositions include (i) a liquid obtained by dispersing a dry powder of a food material containing dietary fiber in water; (ii) a liquid obtained by crushing and dispersing a food material containing dietary fiber in water and removing unnecessary materials as necessary by any means such as centrifugation, filtration, a filter bag, or a sieve; (iii) a liquid obtained by removing components other than dietary fiber from the liquid (i) or (ii) above to increase the dietary fiber content; and (iv) a liquid obtained by mixing a dry powder prepared from any of the liquids (i) to (iii) above with water. A preferred example of a dietary fiber-containing liquid composition includes plant-based milk.

Examples of food materials containing dietary fiber include beans such as soybeans, peas, lentils, chickpeas, black beans, broad beans, mung beans, lupine beans, and kidney beans; grains such as wheat, barley, oats, sorghum, rice, rye, buckwheat, barnyard millet, foxtail millet, teff, corn, and potatoes; nuts such as almonds, coconuts, peanuts, cashew nuts, hazelnuts, pecan nuts, macadamia nuts, pistachios, walnuts, Brazil nuts, pili nuts, chestnuts, sesame seeds, and pine nuts; and seeds such as hemp seeds (industrial hemp), chia seeds, chia, amaranth, canary seeds, and linseed.

The amount of water-soluble dietary fiber in the plant-based dietary fiber-containing composition is not particularly limited, but the lower limit is, for example, 0.01% by weight or more, 0.05% by weight or more, 0.1% by weight or more, preferably 0.25% by weight or more, 0.5% by weight or more, more preferably 1.0% by weight or more, 1.2% by weight or more. The upper limit of the dietary fiber content in the plant-based dietary fiber-containing liquid composition is, for example, 50% by weight or less, 40% by weight or less, 35% by weight or less, preferably 30% by weight or less, 25% by weight or less, more preferably 20% by weight or less, 15% by weight or less.

By treating the dietary fiber-containing compositions exemplified above with the enzyme preparation according to the present technique described above, it is possible to increase the amount of water-soluble dietary fiber in the plant-based dietary fiber-containing liquid composition and reduce the amount of residues.

In addition, details of the enzyme preparation used in the water-soluble dietary fiber increasing agent and the residue reducing agent according to the present technique have been described above, so a description thereof will be omitted here.

3. Dietary fiber-containing processed foods and drinks

The enzyme preparation, the agent for increasing the amount of water-soluble dietary fiber, or the residue-reducing agent according to the present technique can be suitably used in the production of dietary fiber-containing processed foods and drinks. Dietary fiber-containing processed foods and drinks produced using the enzyme preparation, the agent for increasing the amount of water-soluble dietary fiber, or the residue-reducing agent according to the present technique are characterized by having less residue and a larger amount of water-soluble dietary fiber than dietary fiber-containing processed foods and drinks produced without using these agents.

The amount of water-soluble dietary fiber in dietary fiber-containing processed foods and drinks is not particularly limited depending on the raw materials and types of the foods and drinks, but the lower limit is, for example, 0.01% by weight or more, 0.05% by weight or more, 0.1% by weight or more, preferably 0.25% by weight or more, 0.5% by weight or more, and more preferably 1.0% by weight or more. The upper limit of the amount of water-soluble dietary fiber in dietary fiber-containing processed foods and drinks is, for example, 50% by weight or less, 40% by weight or less, 35% by weight or less, preferably 30% by weight or less, 25% by weight or less, and more preferably 20% by weight or less, 15% by weight or less.

Specific examples of dietary fiber-containing processed foods and drinks according to the present technique include plant-based milks such as oat milk (also called "oats milk") and rice milk, vegetable juice, fruit juice, plant-based yogurt, etc.

4. Method for producing a dietary fiber-containing liquid processed composition

The method for producing a dietary fiber-containing liquid processed composition according to the present technique includes a cellulase treatment step. In addition, in the present technique, for example, a liquefaction step, a recovery step, etc. can be performed according to the physical properties of the target dietary fiber-containing liquid processed composition. Each step will be described in detail below.

(1) Cellulase treatment step

The cellulase treatment step is a step of treating a dietary fiber-containing composition (which may be a dietary fiber-containing liquid processed composition) with two or more cellulases having different origins. The details of the cellulase and dietary fiber-containing composition used are as described above, so a description thereof will be omitted here.

Various conditions in the cellulase treatment step can be freely set as long as they do not impair the effects of the present technique. For example, the pH, temperature, action time, etc. can be set according to the physicochemical properties of the cellulase used, such as the optimum pH, stable pH range, optimum temperature, and temperature stability. The pH can be set, for example, to pH 4.0 to 9.5, pH 5.0 to 8.5, preferably pH 5.5 to 7.5, and more preferably pH 6.0 to 7.0. The temperature can be set, for example, to 10°C to 80°C, preferably 25°C to 75°C, 35°C to 70°C, and more preferably 55°C to 65°C. The action time can be set, for example, to 5 minutes to 48 hours, preferably 30 minutes to 24 hours, 1 to 12 hours, 2 to 8 hours, and more preferably 3 to 5 hours. The optimal reaction conditions can be determined through preliminary experiments.

(2) Liquefaction step

The liquefaction step is a step of liquefying the dietary fiber-containing composition. In the present technique, the liquefaction step is not essential, but may be carried out as appropriate when the dietary fiber-containing composition as the raw material contains a component that needs to be liquefied, such as starch. The liquefaction method in the liquefaction step is not particularly limited, and one or a combination of two or more general liquefaction methods can be used. For example, a method using a liquefying enzyme such as $\alpha$-amylase can be mentioned.

The order of the cellulase treatment step and the liquefaction step is not particularly limited. The liquefaction step can be performed before or after the cellulase treatment step, or the cellulase treatment step and the liquefaction step can be performed simultaneously. In the present technique, it is preferable to perform the cellulase treatment step after the liquefaction step.

(3) Recovery step

The recovery step is a step of recovering the dietary fiber-containing liquid processed composition produced through a cellulase treatment step and, if necessary, a liquefaction step. As for the specific recovery method, one or more of the recovery methods generally used in the production of dietary fiber-containing liquid processed composition can be freely combined depending on the type of dietary fiber-containing liquid processed composition to be produced.

The dietary fiber-containing liquid processed composition produced through the cellulase treatment step is characterized by having a reduced amount of residue and an increased amount of water-soluble dietary fiber compared to a dietary fiber-containing liquid processed composition produced without the cellulase treatment step. That is, in the recovery step in the present technique, a dietary fiber-containing liquid processed composition with reduced residue and/or a dietary fiber-containing liquid processed composition with an increased amount of water-soluble dietary fiber can be recovered.

One embodiment of the method for producing a dietary fiber-containing liquid processed composition according to the present technique includes the following steps (1) and (2). Note that an enzyme inactivation step may be added after step (2).

(1) A step of preparing a dietary fiber-containing composition
(2) A step of treating the prepared dietary fiber-containing composition with two or more types of cellulases having different origins.

One embodiment of the method for producing a dietary fiber-containing liquid processed composition according to the present technique includes the following steps (1) to (4). Note that an enzyme inactivation step may be added after steps (2) and/or (3).

(1) A step of preparing a dietary fiber-containing composition
(2) A step of treating the prepared dietary fiber-containing composition with a liquefying enzyme such as $\alpha$-amylase.
(3) A step of treating the dietary fiber-containing composition treated with the liquefying enzyme with two or more types of cellulases having different origins.
(4) A step of recovering the dietary fiber-containing liquid processed composition

One embodiment of the method for producing a plant-based food and drink or a plant-based food and drink material according to the present technique includes the following steps (1) to (3). Note that an enzyme inactivation step may be added after step (2).

(1) A step of preparing a dietary fiber-containing composition
(2) A step of adding a liquefying enzyme such as $\alpha$-amylase to the prepared dietary fiber-containing composition and treating it with two or more types of cellulases having different origins.
(3) A step of recovering the dietary fiber-containing liquid processed composition

The enzymes in step (2) may be used for treatment treated simultaneously or separately. When the enzyme treatments are performed separately, the order of the treatments is not particularly limited. When the enzyme treatments are performed separately, an enzyme inactivation step may be added between each enzyme treatment as necessary.

5. Method for increasing the amount of water-soluble dietary fiber in a dietary fiber-containing liquid processed composition and method for reducing residues

The method for increasing the amount of water-soluble dietary fiber in the dietary fiber-containing liquid processed composition and the method for reducing residues in the dietary fiber-containing liquid processed composition according to the present technique are methods that include a cellulase treatment step. In addition, in the present technique, for example, a liquefaction process, a recovery process, etc. can also be performed according to the physical properties of the target dietary fiber-containing liquid processed composition. The details of each process are as described above, so the explanation will be omitted here.

The present technique can have the following configurations.

(1) An enzyme preparation containing two or more types of cellulase having different origins.
(2) The enzyme preparation according to (1), which contains exo-type cellulase and endo-type cellulase.
(3) The enzyme preparation according to (2), wherein the exo-type cellulase is a cellulase derived from a microorganism of the genus Trichoderma.
(4) The enzyme preparation according to (2) or (3), wherein the endo-type cellulase is a cellulase derived from a microorganism of the genus Aspergillus.
(5) An agent for increasing the amount of water-soluble dietary fiber in a dietary fiber-containing liquid processed composition, comprising the enzyme preparation according to any one of (1) to (4).
(6) The agent according to (5), comprising exo-type cellulase and endo-type cellulase, wherein the activity ratio of the exo-type cellulase to the endo-type cellulase is exo-type cellulase activity:endo-type cellulase activity = 1:99 to 99:1.
(7) A residue-reducing agent in a dietary fiber-containing liquid processed composition, comprising the enzyme preparation according to any one of (1) to (4).
(8) The residue-reducing agent according to (7), comprising exo-type cellulase and endo-type cellulase, wherein the activity ratio of the exo-type cellulase to the endo-type cellulase is exo-type cellulase activity:endo-type cellulase activity = 1:99 to 99:1.

(9) A dietary fiber-containing processed food and drink containing dietary fiber, in which the enzyme preparation according to any one of (1) to (4) is used.

(10) A method for producing a dietary fiber-containing liquid processed composition, comprising a cellulase treatment step of treating a dietary fiber-containing composition with two or more types of cellulase having different origins.

(11) The method for producing a dietary fiber-containing liquid processed composition according to (10), wherein the dietary fiber-containing composition is a cereal.

(12) The method for producing a dietary fiber-containing liquid processed composition according to (10) or (11), wherein the two or more types of cellulases having different origins contain exo-type cellulase and endo-type cellulase.

(13) The method for producing a dietary fiber-containing liquid processed composition according to (12), wherein the exo-type cellulase is a cellulase derived from a microorganism of the genus Trichoderma.

(14) The method for producing a dietary fiber-containing liquid processed composition according to (12) or (13), wherein the endo-type cellulase is a cellulase derived from a microorganism of the genus Aspergillus.

(15) The method for producing a dietary fiber-containing liquid processed composition according to any one of (12) to (14), wherein in the cellulase treatment step, the exo-type cellulase and the endo-type cellulase are allowed to act in an activity ratio of exo-type cellulase activity:endo-type cellulase activity = 1:99 to 99:1.

(16) A method for increasing the amount of water-soluble dietary fiber in a dietary fiber-containing liquid processed composition, comprising a cellulase treatment step of treating the dietary fiber-containing liquid processed composition with two or more types of cellulase having different origins.

(17) A method for reducing residues in a dietary fiber-containing liquid processed composition, comprising a cellulase treatment step of treating the dietary fiber-containing liquid processed composition with two or more types of cellulase having different origins.

EXAMPLES

The present invention will be described in more detail below with reference to examples. Note that the examples described below are representative examples of the present invention, and the scope of the present invention should not be construed as being narrowed by these examples.

1. Raw material

The enzymes and plant-based raw materials used in the examples are shown in Table 1 below.

[Table 1]

| Enzyme/plant-based raw material | Product name | Sold by |
|---|---|---|
| $\alpha$-amylase | Bacillus amyloliquefaciens-derived $\alpha$-amylase | Amano Enzyme Inc. |
| Cellulase 1 | Trichoderma viride-derived cellulase | Amano Enzyme Inc. |
| Cellulase 2 | Aspergillus niger-derived cellulase | Amano Enzyme Inc. |
| Oat flour | Premium oat flour<br>Protein content 13.1 weight%, carbohydrate content 67.7 weight%, dietary fiber content 10.1 weight% | Slow Food |

2. Enzyme activity measurement method

[Method for measuring cellulase activity]

The cellulase activity can be measured by a method based on the cellulase activity test method in the 9th edition of the Japan's Specifications and Standards for Food Additives.

About 1 g of carmellose sodium (also known as sodium carboxymethylcellulose) (degree of etherification 0.62-0.68) was precisely weighed out in advance, and dried at 105°C for 4 hours to measure the weight loss. Carmellose sodium equivalent to 0.625 g of the dried product was precisely weighed out and placed in a 100 mL Erlenmeyer flask, 50 mL of water was added, and the mixture was heated to dissolve, and after cooling, 10 mL of 1 mol/L acetic acid-sodium acetate buffer (pH 4.5) and water were added to make exactly 100 mL to prepare the substrate solution.

4 mL of substrate solution was accurately measured and placed in a 50 mL Nessler tube, and left at $37\pm0.5°C$ for 10 minutes or more, after which 1 mL of sample solution was accurately measured and added, and immediately shaken. This solution was left at $37\pm0.5°C$ for accurately 30 minutes, 2 mL of alkaline copper test solution was accurately added and shaken, the Nessler tube was plugged, and heated in a water bath for 30 minutes. After cooling with water, 2 mL of Nelson's solution was accurately added and shaken well, and 3 mL of 0.5 mol/L sodium hydroxide test solution was accurately added, shaken to dissolve the precipitate, left for 20 minutes, and then 13 mL of 1 mol/L acetic acid/sodium acetate buffer (pH 4.5) was accurately added. 1 mL of this solution was accurately measured, 9 mL of 1 mol/L acetic acid/sodium acetate buffer (pH 4.5) was accurately added, and shaken well. The absorbance (A1) of this solution at a wavelength of 750 nm was measured using water as a control.

Separately, 1 mL of sample solution was accurately measured and placed in a 50 mL Nessler tube, 2 mL of alkaline copper test solution was accurately added and shaken, 4 mL of substrate solution was then accurately measured and added, shaken, and the absorbance (A2) was measured in the same manner.

Under these conditions, regarding cellulase activity, the amount of enzyme that brings about an increase in reducing power equivalent to 1 $\mu$mole of glucose per minute is defined as 1 unit, and cellulase activity per 1 g or 1 mL of sample (U/g, U/mL) was calculated using the following formula.

$$\text{Cellulase activity (U/g, U/mL)} = G \times (1/30) \times (1/0.180) \times n$$

G: Amount of glucose produced (mg) calculated from the glucose calibration curve using the (A1-A2/a) value
1/30: Conversion factor per minute
1/0.180: 1 $\mu$mole of glucose = 0.180 mg
a: Slope of glucose calibration curve
n: Dilution factor per 1 g or 1 mL of sample

[Method for measuring $\alpha$-amylase activity]

10 mL of 1% potato starch substrate solution (0.1 mol/L acetic acid (pH 5.0)) was heated at 37°C for 10 minutes, and then 1 mL of sample solution containing $\alpha$-amylase was added and immediately shaken. After leaving this solution at 37°C for 10 minutes, 1 mL of this solution was added to 10 mL of 0.1 mol/L hydrochloric acid test solution and immediately shaken. Next, 0.5 mL of this solution was measured, 10 mL of 0.0002 mol/L iodine test solution (Japanese Pharmacopoeia) was added, and after shaking, the absorbance (AT) at a wavelength of 660 nm was measured using water as a control. Separately, 1 mL of water was added instead of the sample solution and the same procedure was performed to measure the absorbance (AB). Under these conditions, the activity of $\alpha$-amylase was calculated from the following formula, with the amount of enzyme that reduces the color of potato starch by iodine by 10% in 1 minute being 1 unit (1U).

$$\alpha\text{-Amylase activity (U/g, U/mL)} = \{(AB-AT)/AB\} \times 1/W$$

AT: absorbance of reaction solution
AB: absorbance of blank solution
W: Amount of sample in 1 mL of sample solution (g or mL)

3. Experimental example

<Experimental Example 1>

In Experimental Example 1, the effect of the enzyme used on the amount of residue in the dietary fiber-containing liquid processed composition was examined. In Experimental Example 1, oat flour was used as an example of a dietary fiber-containing composition, and processed oat milk was produced as an example of a dietary fiber-containing liquid processed composition. In addition, the content of cellooligosaccharide was quantified as an example of water-soluble dietary fiber.

(1) Production of processed oat milk

18.1 g of oat flour was mixed with 150 g of water and suspended in a 300 mL Erlenmeyer flask. 0.01% $\alpha$-amylase was added to this suspension and reacted at 60°C for 0.5 hours. Then, enzymes were added in the combinations shown in Table 2 below and reacted at 60°C for 2 or 4 hours. After the reaction, the mixture was heated at 95°C for 5 minutes to inactivate the enzyme, and processed oat milk was produced.

(2) Measurement and Evaluation

[Residue reduction rate]

The produced processed oat milk was centrifuged (9,000×g, 5 minutes) and the supernatant was removed. The resulting residue was dried at 80°C for 6 hours to obtain a dry residue.
The amount of the dried residue obtained was measured as actual measurement data, and the ratio to the amount of the residue in the control example was calculated, and the residue reduction rate was calculated using the following formula.

Residue reduction rate = 100-{(residue amount of each comparative example or each example/residue amount of control example)× 100}

As theoretical data, the theoretical value of the residue reduction rate was calculated from the dry residue rates of Comparative Examples 1 and 2 and the blending ratio of the enzymes in each Example.

[Quantitative Determination of Water-Soluble Dietary Fiber Amount]

Glucoamylase with a final concentration of 1.0 mg/mL was added to the processed oat milk produced by cellulase treatment for 4 hours, and reacted at 50°C for 2 hours. The resulting reaction solution was heated at 95°C for 5 minutes to inactivate the enzyme. After inactivation, 190 μL of 1 mol/L trichloroacetic acid was added to 1.5 mL of the enzyme-inactivated oat solution, and the mixture was left at room temperature for 10 minutes. After 10 minutes, the mixture was centrifuged (12,000 rpm, 10 minutes) to collect the supernatant. An equal amount of diethyl ether was added to the collected supernatant to suspend it. The mixture was centrifuged (12,000 rpm, 10 minutes), the organic solvent layer was discarded, and the aqueous layer was collected. This operation was repeated twice. An equal amount of 99.5% ethanol (for high performance liquid chromatography, Fujifilm Wako Pure Chemical Corp.) was added to the collected aqueous layer, stirred, and filtered through a membrane filter, which was then subjected to analysis. The analysis was performed using high performance liquid chromatography, and the column used was an MCL GEL CK04S manufactured by Mitsubishi Chemical Corp. From the peak areas, the total amount of each cellooligosaccharide (C2: cellobiose, C3: cellotriose, C4: cellotetraose, C5: cellopentaose, C6: cellohexaose) produced was evaluated as the amount of water-soluble dietary fiber produced.

(3) Results

The results are shown in Table 2.

[Table 2]

| | | | Control Example | Comparative Example | Example | | | Comparative Example |
|---|---|---|---|---|---|---|---|---|
| | | | 1 | 1 | 1 | 2 | 3 | 2 |
| Cellulase 1 | | | 0 | 0 (0) | 3.5 (25) | 7 (50) | 10.5 (75) | 14 (100) |
| Cellulase 2 | | U/g-fiber | 0 | 74 (100) | 55.5 (75) | 37 (50) | 18.5 (25) | 0 (0) |
| Total activity | | | 0 | 74 | 59 | 44 | 29 | 14 |
| Cellulase 1 : Cellulase 2 | | | - | 0:100 | 6:94 | 16:84 | 36:64 | 100:0 |
| Residue reduction rate | Cellulase treatment time — 2 hours | Actual measurement (%) | 0 | 7.5 | 11.8 | 23.4 | 26.0 | 23.0 |
| | | Theoretical value(%) | 0 | 7.5 | 11.4 | 15.2 | 19.1 | 23.0 |
| | | Actual measurement/theoretical value | 0 | 1.00 | 1.04 | 1.54 | 1.36 | 1.00 |
| | 4 hours | Actual measurement (%) | 0 | 10.9 | 19.5 | 27.0 | 36.3 | 29.6 |
| | | Theoretical value(%) | 0 | 10.9 | 15.6 | 20.2 | 24.9 | 29.6 |
| | | Actual measurement/theoretical value | 0 | 1.00 | 1.25 | 1.34 | 1.46 | 1.00 |
| Amount of water-soluble dietary fiber produced | | Actual measurement (mg/mL) | 0.33 | 3.46 | 3.51 | 3.63 | 3.93 | 3.46 |
| | | Theoretical value (mg/mL) | - | 3.46 | 3.46 | 3.46 | 3.46 | 3.46 |
| | | Actual measurement/theoretical value | - | 1.00 | 1.01 | 1.05 | 1.14 | 1.00 |

The numbers in parentheses for Cellulase 1 indicate the relative values of the addition activity of Examples 1 to 3 when the addition activity of Comparative Example 2 is taken as 100.

The numbers in parentheses for Cellulase 2 indicate the relative values of the addition activity of Examples 1 to 3 when the addition activity of Comparative Example 1 is taken as 100.

The amounts of cellulase 2 in Comparative Example 1 and cellulase 1 in Comparative Example 2 added were set so that the amounts of water-soluble dietary fiber produced were the same. Since the amounts of water-soluble dietary fiber produced in Comparative Examples 1 and 2 are the same, the theoretical values of the amounts of water-soluble dietary fiber produced in each Example are also the same as those in Comparative Examples 1 and 2.

(4) Discussion

As shown in Table 2, the actual measured values of the residue reduction rates in Examples 1 to 3 all exceeded the theoretical values.

These results show that the use of two or more types of cellulases from different origins synergistically reduces residues in a dietary fiber-containing liquid processed composition to a degree greater than a person skilled in the art could expect.

In addition, the actual measured values of the amounts of water-soluble dietary fiber produced in Examples 1 to 3 all exceeded the theoretical values. These results show that the use of two or more types of cellulases having different origins synergistically increases the amount of water-soluble dietary fiber in the dietary fiber-containing liquid processed composition to a degree greater than a person skilled in the art could expect.

**Claims**

1. An enzyme preparation comprising two or more types of cellulase having different origins.

2. The enzyme preparation according to claim 1, comprising exo-type cellulase and endo-type cellulase.

3. The enzyme preparation according to claim 2, wherein the exo-type cellulase is a cellulase derived from a microorganism of the genus Trichoderma.

4. The enzyme preparation according to claim 2, wherein the endo-type cellulase is a cellulase derived from a microorganism of the genus Aspergillus.

5. An agent for increasing the amount of water-soluble dietary fiber in a dietary fiber-containing liquid processed composition, comprising the enzyme preparation according to any one of claims 1 to 4.

6. The increasing agent according to claim 5, comprising exo-type cellulase and endo-type cellulase, wherein the activity ratio of the exo-type cellulase to the endo-type cellulase is exo-type cellulase activity:endo-type cellulase activity = 1:99 to 99:1.

7. An agent for reducing residues in a dietary fiber-containing liquid processed composition, comprising the enzyme preparation according to any one of claims 1 to 4.

8. The residue-reducing agent according to claim 7, comprising exo-type cellulase and endo-type cellulase, wherein the activity ratio of the exo-type cellulase to the endo-type cellulase is exo-type cellulase activity:endo-type cellulase activity = 1:99 to 99:1.

9. A dietary fiber-containing processed food and drink, in which the enzyme preparation according to any one of claims 1 to 4 is used.

10. A method for producing a dietary fiber-containing liquid processed composition, comprising a cellulase treatment step of treating a dietary fiber-containing composition with two or more types of cellulase having different origins.

11. The method for producing a dietary fiber-containing liquid processed composition according to claim 10, wherein the dietary fiber-containing composition is a grain.

12. The method for producing a dietary fiber-containing liquid processed composition according to claim 10, wherein the two or more types of cellulases having different origins contain exo-type cellulase and endo-type cellulase.

13. The method for producing a dietary fiber-containing liquid processed composition according to claim 12, wherein the exo-type cellulase is a cellulase derived from a microorganism of the genus Trichoderma.

14. The method for producing a dietary fiber-containing liquid processed composition according to claim 12, wherein the endo-type cellulase is a cellulase derived from a microorganism of the genus Aspergillus.

15. The method for producing a dietary fiber-containing liquid processed composition according to claim 12, wherein in the cellulase treatment step, the exo-type cellulase and the endo-type cellulase are allowed to act in an activity ratio of exo-type cellulase activity:endo-type cellulase activity = 1:99 to 99:1.

16. A method for increasing the amount of water-soluble dietary fiber in a dietary fiber-containing liquid processed composition, comprising a cellulase treatment step of treating the dietary fiber-containing liquid processed composition with two or more types of cellulase having different origins.

17. A method for reducing residues in a dietary fiber-containing liquid processed composition, comprising a cellulase treatment step of treating the dietary fiber-containing liquid processed composition with two or more types of cellulase having different origins.

## INTERNATIONAL SEARCH REPORT

| | International application No. |
|---|---|
| | **PCT/JP2024/002237** |

**A. CLASSIFICATION OF SUBJECT MATTER**

*C12N 9/42*(2006.01)i; *A23L 2/38*(2021.01)i; *A23L 7/104*(2016.01)i
FI: C12N9/42; A23L7/104; A23L2/38 J

According to International Patent Classification (IPC) or to both national classification and IPC

**B. FIELDS SEARCHED**

Minimum documentation searched (classification system followed by classification symbols)

C12N9/42; A23L2/38; A23L7/104

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | HOSHINO, Eiichi et al. Synergistic Actions of Exo-Type Cellulases in the Hydrolysis of Cellulose with Different Crystallinities. Journal of Fermentation and Bioengineering. 1997, vol. 84, no. 4, pp. 300-306, doi:10.1016/S0922-338X(97)89248-3 | 1-4 |
| Y | abstract, MATERIALS AND METHODS, Source of enzymes, RESULTS, tables 3-4 | 2-17 |
| X | WINARSIH, Sri et al. Hydrolysis of corncobs using a mixture of crude enzymes from Trichoderma reesei and Aspergillus niger for bioethanol production. Energy Reports. 2020, vol. 6, pp. 256-262, doi:10.1016/j.egyr.2020.11.141 | 1-4, 10-15 |
| Y | abstract, 2.2 Crude enzyme production, 2.3 Corncobs hydrolysis, 4. Conclusion | 2-17 |
| Y | JP 2002-58449 A (TOYO SHINYAKU CO., LTD.) 26 February 2002 (2002-02-26) claims, paragraphs [0003]-[0005], examples | 5-9, 16-17 |
| Y | JP 2017-132708 A (HYDROX INC.) 03 August 2017 (2017-08-03) claims, paragraph [0025], example 1 | 5-9, 16-17 |

☑ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **12 April 2024** | **23 April 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/002237** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 107821600 A (ZHEJIANG LIZIYUAN FOOD CO., LTD.) 23 March 2018 (2018-03-23) claims, paragraph [0015], examples | 9-15 |

Form PCT/ISA/210 (second sheet) (July 2022)

**INTERNATIONAL SEARCH REPORT**
Information on patent family members

International application No.

**PCT/JP2024/002237**

| Patent document cited in search report | | | Publication date (day/month/year) | Patent family member(s) | Publication date (day/month/year) |
|---|---|---|---|---|---|
| JP | 2002-58449 | A | 26 February 2002 | (Family: none) | |
| JP | 2017-132708 | A | 03 August 2017 | (Family: none) | |
| CN | 107821600 | A | 23 March 2018 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107821600 A
- WO 2006011479 A

- JP 2009095252 A

**Non-patent literature cited in the description**

- *Acta Cryst.*, 2002, vol. D58, 660-667